(19) Europäisches Patentamt
European Patent Office
Office européen des brevets

(11) Veröffentlichungsnummer: **0 043 024**
**B1**

# (12) EUROPÄISCHE PATENTSCHRIFT

(45) Veröffentlichungstag der Patentschrift: **28.03.84**

(51) Int. Cl.³: **C 07 D 261/14**

(21) Anmeldenummer: **81104613.5**

(22) Anmeldetag: **15.06.81**

## (54) Verfahren zur Herstellung von 5-Aminoisoxazolen.

(30) Priorität: **02.07.80 DE 3024989**

(43) Veröffentlichungstag der Anmeldung: **06.01.82 Patentblatt 82/1**

(45) Bekanntmachung des Hinweises auf die Patenterteilung: **28.03.84 Patentblatt 84/13**

(84) Benannte Vertragsstaaten: **AT BE CH DE FR GB IT LI LU NL SE**

(56) Entgegenhaltungen:
**DD - A - 29 481**
**DE - A - 1 695 199**
**DE - A - 1 814 116**

(73) Patentinhaber: **DYNAMIT NOBEL AKTIENGESELLSCHAFT, Postfach 1209, D-5210 Troisdorf, Bez. Köln (DE)**

(72) Erfinder: **Peeters, Hermann, Dr., Porzer Strasse 1, D-5216 Niederkassel-Ranzel (DE)**
Erfinder: **Vogt, Wilhelm, Dr., Neuenhöfer Allee 54, D-5000 Köln (DE)**

**EP 0 043 024 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 5-Aminoisoxazolen

Die Erfindung betrifft die Herstellung von 5-Aminoisoxazolen der Formel

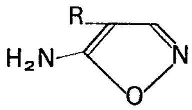

(1)

worin R Wasserstoff oder einen inerten organischen Rest bedeutet, durch Umsetzung von Metallsalzen des den Substituenten R enthaltenden β-Hydroxiacrylnitrils mit Säureadditionssalzen des Hydroxylamins.

Die Herstellung einer Anzahl von 5-Aminoisoxazolen auf verschiedenen Wegen ist bekannt:

Nach der US-PS Nr. 3917632 wird das 3-Dimethylaminoacrylnitril mit Säureadditionssalzen des Hydroxylamins in inerten Lösungsmitteln umgesetzt.

Hydroxylamin kann an Cyanacetylen nach „Chem. pharm. Bl." (Japan), *14*, 1277 (1966) = „C.A.", *67* (1977) 32627 j addiert werden. Die Umsetzung des toxischen Natriumcyanids mit β-Chloracetaldehydoxim ist nach „Tetrahedron Letters" (1969) 4817 möglich. Die Synthese von 5-Amino-4-phenylisooxazol kann durch Reaktion des freien α-Formylphenylacetonitrils mit Hydroxylaminhydrochlorid nach „Arch. Pharm.", *300*, 615 (1967) erfolgen.

Nach diesem Stand der Technik ist im allgemeinen nur die Herstellung ausgewählter Aminoisoxazole, beispielsweise des Grundkörpers oder weniger Derivate mit ganz bestimmten Substituenten in 4-Stellung möglich. Beispielsweise ist die letztgenannte Synthese nur mit dem sehr stabilen α-Formylarylacetonitrilen ausführbar, die zudem vor der Verwendung gesondert in freier Form aus den Salzen herzustellen und zu reinigen sind.

In vielen Fällen sind auch hohe Stoffkosten, umständliche Herstellwege der Ausgangsstoffe, die Verwendung hochtoxischer Substanzen und geringe Ausbeuten nachteilig.

Es bestand daher die Aufgabe, 5-Aminoisoxazol und in 4-Stellung substituierte 5-Aminoisoxazole nach einem für alle Stoffe gleich gut ausführbaren Verfahren herzustellen, so dass die sehr zahlreichen in verschiedenster Weise in 4-Stellung substituierten 5-Aminoisoxazole, die als pharmazeutische Wirkstoffe sowie als Zwischenprodukte zu deren Herstellung dienen, einfach und durch einen einheitlichen Verfahrensweg mit gleichen Reaktionsmitteln in guter Qualität zugängig werden.

Gegenstand der Erfindung ist ein Verfahren zur Herstellung von 5-Aminoisoxazolen der Formel

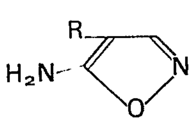

(1)

worin R ein Wasserstoffatom, geradkettige, verzweigte oder cyclische Alkylreste mit 1 bis 20 Kohlenstoffatomen, geradkettige oder verzweigte Reste $-(CH_2)_n-CN$, $-(CH_2)_n-COOR'$, $-(CH_2)_n-NH_2$, $-(CH_2)_n-OR'$, wobei R' ein geradkettiger, verzweigter oder cyclischer Alkylrest oder ein Phenylrest und n = 1 bis 5 ist, oder $(CH_2)_m-Cyc$, wobei Cyc ein isocyclischer oder heterocyclischer Ring mit ein- oder mehrcyclischer Struktur oder ein aromatischer oder heteroaromatischer Ring mit ein- oder mehrcyclischer Struktur und m = 0 bis 5 ist, bedeutet, dadurch gekennzeichnet, dass eine Verbindung der Formel

$$\left(\tfrac{1}{\alpha}\,Me\right) O - CH = \underset{\displaystyle R}{\underset{|}{C}} - CN \qquad (2)$$

wobei R dieselbe Bedeutung wie in der Formel 1 hat und Me ein Alkalimetall mit α = 1 oder ein Erdalkalimetall mit α = 2 ist, mit einem Säureadditionssalz des Hydroxylamins der Formel

$$H_2N\,OH \cdot HX \qquad (3)$$

wobei HX eine ein- oder mehrbasische anorganische oder organische Säure bedeutet, bei Temperaturen von −10°C bis 80°C und Drücken zwischen 1 und 5 bar umgesetzt wird.

Besonders vorteilhaft und daher bevorzugt ist das Verfahren für Substituenten R, die nicht Arylreste sind.

Die Synthese von 5-Amino-4-phenylisoxazol gemäss der Erfindung ist gegenüber der Synthese mit freien α-Formylacetonitrilen dadurch entscheidend vereinfacht, dass das bei der Formylierung substituierter Acetonitrile primär entstehende Metallsalz des jeweiligen α-Formylacetonitrils beispielsweise nach der deutschen Offenlegungsschrift Nr. 2753322.8, direkt eingesetzt werden kann und der aufwendige Verfahrensschritt zur Isolierung des freien Aldehyds und seine Reinigung eingespart wird. Es hat sich nämlich gezeigt, dass lediglich die sehr stabilen α-Formylarylacetonitrile als freie Verbindungen erhalten werden können, während aliphatisch oder araliphatisch substituierte α-Formylacetonitrile und solche mit weiteren nicht aromatischen Substituenten nicht unzersetzt als freie Verbindungen erhältlich sind. Selbst im Fall guter Stabilität ist aber die Freisetzung aus den Metallsalzen nachteilig, da Verluste und Verunreinigungen auftreten bzw. eine zusätzliche Reinigung erforderlich ist.

Die Erfindung löst demgemäss die gestellte Aufgabe, auf vereinfachte Weise alle 5-Aminoisoxazole durch einen gleichartigen Verfahrensweg und in gleichmässig sehr hoher Ausbeute, unabhängig von der Art des Substituenten in 4-Stellung, zugängig zu machen. Das Verfahren überrascht durch seine einfache Ausführungsform unter milden Bedingungen und die Abwesenheit von Hilfsstoffen bei der Reaktion. Die Metallsalze bieten zudem den Vorteil einfach anorganischer Salze als einziges Nebenprodukt und der Steuerung des pH-Wertes in den Bereich oberhalb pH 5, der zur Cyclisierung notwendig ist. Unter den Metallsalzen sind die Natriumsalze bevorzugt.

Die Metallsalze bieten den weiteren Vorteil, ohne vorangehende Reinigung verwendbar zu sein. Damit ist die vorher schwierige Synthese der 5-Aminoisoxazole letztlich auf die Herstellung aus einfachen, den 4-Substituenten enthaltenden Nitrilen zurückgeführt.

Das erfindungsgemässe Verfahren kann so durchgeführt werden, dass die Metallsalze von 3-Hydroxiacrylnitrilen der Formel (2) als Feststoff, Suspension oder gegebenenfalls Lösung zu der Suspension oder Lösung des Säureadditionssalzes des Hydroxylamins (3) zugegeben werden. Auch kann das Metallsalz vorgelegt und das Säureadditionssalz des Hydroxylamins zugegeben werden oder beide Salze werden gleichzeitig zur Reaktionslösung gegeben, doch ist es im allgemeinen wegen der Instabilität der meisten Metallsalze von 3-Hydroxiacrylnitrilen der Formel (2) in Lösung günstiger, das Metallsalz portionsweise zuzugeben. Alle bekannten Säureadditionssalze des Hydroxylamins können bei der erfindungsgemässen Reaktion zur Anwendung kommen, von denen die im Handel befindlichen Salze der Halogenwasserstoffsäuren, insbesondere der Salzsäure, und die Salze der Schwefelsäure bevorzugt sind.

Die Reaktion wird im allgemeinen in einem polaren Lösungsmittel durchgeführt so z.B. in Wasser oder Alkoholen in polar aprotischen Lösungsmitteln, wie Dimethylformamid oder Dimethylsulfoxid. Gegebenenfalls können auch unpolare Lösungsmittel wie Toluol oder Gemische von Lösungsmitteln Verwendung finden. Besonders geeignet sind Wasser und Alkohole mit 1 bis 3 Kohlenstoffatomen.

Die Reaktionstemperatur kann zwischen −10°C und +80°C liegen. Bevorzugt sind Temperaturen zwischen +10°C und 40°C. Zur Abführung der Reaktionswärme beim Zusammengeben ist bei Einsatz einiger Metallsalze von 3-Hydroxiacrylnitrilen Kühlung erforderlich. Der Druck ist nicht kritisch und kann zwischen 1 und 5 bar, vorzugsweise bei Normaldruck liegen. Die Reaktion ist im allgemeinen in 0,5 bis 5 h beendet.

Die Reaktanten werden in etwa äquimolaren Mengen zusammengegeben. Der Überschuss einer Komponente ist möglich, sollte aber 20% nicht überschreiten. Die Konzentration der Reaktionspartner sollte möglichst hoch sein, die Grenzen werden durch die Löslichkeiten der Salze bzw. durch die gute Rührbarkeit der Reaktionsmischung gegeben. Ein Katalysator oder ein basisches Kondensationsmittel ist nicht notwendig. Gegebenenfalls können jedoch basische Kondensationsmittel wie z.B. Alkoholate oder Alkali- bzw. Erdalkalihydroxyde Verwendung finden. Diese oder eine kleine Menge Alkalihydroxid sind auch zweckmässig, sofern die Reaktionsteilnehmer allein einen pH-Wert unterhalb 5 bewirken, da bei zu kleinen pH-Werten nicht cyclische Produkte entstehen können.

Die Aufarbeitung des Reaktionsansatzes kann durch Abfiltrieren des gebildeten Reaktionsproduktes aus der Reaktionslösung oder durch Extraktion mit geeigneten Extraktionsmitteln wie Essigester erfolgen.

Vor der Filtration oder Extraktion kann ein Teil der Lösungsmittel oder eine Komponente im Falle eines Lösungsmittelgemisches oder das gesamte Lösungsmittel, z.B. durch Destillation im Vakuum, entfernt werden.

5-Aminoisoxazole der Formel (1) finden Verwendung als Zwischenprodukt zur Arzneimittelsynthese und in organischen Synthesen, so für den Ataracticum (DE-OS Nr. 2215087), für ein Bactericid (US-PS Nr. 3468900) und zur Darstellung von Cyanacetamid (US-PS Nr. 3917632). Weiter können 5-Aminoisoxazole u.a. zu Substanzen mit antiinflammatorischen Eigenschaften (US-PS Nr. 3891630) und Stoffen mit trichomonacitaler Wirkung (113, 26 = „C.A.", *81*, [1974] 49600 s) umgesetzt sowie unter Abspaltung von R mit Pt-Katalysatoren („J. Org. Chem.", *42*, [1977] 109) ringöffnend hydriert werden.

*5-Aminoisoxazol*

*Beispiel 1:*

Zu einer Suspension von 59 g (0,825 mol) Hydroxyaminhydrochlorid in 200 ml Methanol werden unter Kühlung bei 18 bis 20°C innerhalb von 2 h portionsweise 87 g (0,75 mol) Natrium-3-hydroxiacrylnitril (Gehalt 78,6%) zugegeben und die Mischung 3 h bei Raumtemperatur gerührt. Der Rückstand nach Abziehen des Lösungsmittels wird in 100 ml Wasser aufgenommen und mit Essigester extrahiert. Nach dem Trocknen und Abziehen des Essigesters werden 61,8 g (98% d. Th.) 5-Aminoisoxazol erhalten. Schmp.: 72-73°C.

$^1$H-NMR-Spektrum (DMSO-$d_6$): δ (ppm) = 4,94 (d, J = 1,4 Hz, CH), 6,59 (s. breit, $NH_2$), 8,00 (d, J = 1,4 Hz, −CH=N−).

*Beispiel 2:*

Zu einer Suspension von 14,6 g (0,21 mol) Hydroxylaminhydrochlorid in 50 ml Toluol/Methanol 1:3 werden unter Kühlung auf ≤ 20°C innerhalb von 60 min portionsweise 23,2 g (0,20 mol) Natrium-3-hydroxiacrylnitril (Gehalt 78,6%) zugegeben und die Mischung 2 h bei Raumtemperatur gerührt. Nach Aufarbeitung wie im Beispiel 1 werden 15,4 g (91,7% d. Th.) 5-Aminoisoxazol erhalten. Schmp.: 68-70°C. Das $^1$H-NMR-Spektrum entspricht dem in Beispiel 1.

*Beispiel 3:*

Zu einer Lösung von 7,6 g (0,11 mol) Hydroxylaminhydrochlorid in 20 ml Wasser werden unter Kühlung auf ≤ 20°C innerhalb von 40 min 11,8 g (0,1 mol) Natriumhydroxiacrylnitril (Gehalt 77,1%) portionsweise zugegeben und die Lösung 1 h bei Raumtemperatur gerührt. Das Reaktionsgemisch wird mit Essigester extrahiert und das Lösungsmittel wird nach dem Trocknen abgezogen. Es werden 7,7 g (91,6% d. Th.) 5-Aminoisoxazol erhalten. Schmp.: 72°C. Das $^1$H-NMR-Spektrum entspricht dem in Beispiel 1.

*Beispiel 4:*

Zu einer Lösung von 9 g (0,055 mol) Hydroxylaminsulfat in 25 ml Wasser werden unter Kühlung auf 20°C innerhalb von 30 min 11,8 g (0,1 mol) Natriumhydroxiacrylnitril (Gehalt 77,1%) portionsweise zudosiert und die Lösung 3 h bei Raumtemperatur gehalten. Die Reaktionslösung wird mit Essigester extrahiert und das Lösungsmittel wird nach dem Trocknen abgezogen. Es werden 7,4 g (88,1% d. Th.) 5-Aminoisoxazol erhalten. Schmp.: 69-70°C. Das $^1H$-NMR-Spektrum entspricht dem in Beispiel 1.

*5-Amino-4-methylisoxazol*

*Beispiel 5:*

Zu einer Suspension von 2,5 g (0,037 mol) Hydroxylaminhydrochlorid in 10 ml Methanol werden unter Kühlung auf 20°C innerhalb von 20 min 5 g (0,033 mol) Natrium-3-hydroxi-2-methylacrylnitril (Gehalt 70%) portionsweise zudosiert und die Mischung 3 h bei Raumtemperatur gerührt. Nach Aufarbeitung wie im Beispiel 1 werden 2,5 g (77,3% d. Th.) 5-Amino-4-methylisoxazol erhalten. Schmp.: 61°C. $^1H$-NMR-Spektrum ($CDCl_3$): δ (ppm) = 1,82 (s. 3, $CH_3$), 5,40 (s, breit 2, $NH_2$), 7,91 (s. 1, HC=).

*Beispiel 6:*

Zu einer Suspension von 5,2 g (0,075 mol) Hydroxylaminhydrochlorid in 50 ml Methanol werden unter Kühlung auf <20°C innerhalb von 20 min 17,9 g (0,075 mol) Natrium-3-hydroxi-2-benzylacrylnitril (Gehalt 76%) portionsweise zudosiert und die Mischung 3 h bei Raumtemperatur gerührt. Nach Aufarbeitung wie im Beispiel 1 werden 11,0 g (84,3% d. Th.) 5-Amino-4-benzylisoxazol erhalten. Schmp.: 125-128°C Molekülmasse (massenspektroskopisch) 174.

*5-Amino-4-phenylisoxazol*

*Beispiel 7:*

Zu einer Suspension von 2,5 g (0,037 mol) Hydroxylaminhydrochlorid in 10 ml Methanol werden unter Kühlung bei 18°C 5,95 g (0,033 mol) Natrium-3-hydroxy-2-phenylacrylnitril (Gehalt 92,6%) innerhalb von 15 min portionsweise zudosiert und die Mischung 3 h bei Raumtemperatur gerührt. Nach Abtrennen des Natriumchlorids wird der Methanol abgezogen. Der Rückstand wird mit Essigester extrahiert, die Lösung getrocknet und das Lösungsmittel abgezogen. Es werden 5,2 g (98,5% d. Th.) 5-Amino-4-phenylisoxazol erhalten. Schmp.: 96°C.

$^1H$-NMR- (DMSO-$d_6$): δ (ppm) = 7,13-7,76 (m, 7, $C_6H_5$ und $NH_2$), 8,71 (S, 1, H-C).

**Patentansprüche**

1. Verfahren zur Herstellung von 5-Aminoisoxazolen der Formel

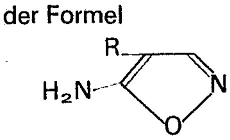

(1)

worin R ein Wasserstoffatom, geradkettige, verzweigte oder cyclische Alkylreste mit 1 bis 20 Kohlenstoffatomen, geradkettige oder verzweigte Reste $-(CH_2)_n-CN$, $-(CH_2)_n-COOR'$, $-(CH_2)_n-NH_2$, $-(CH_2)_n-OR'$, wobei R' ein geradkettiger, verzweigter oder cyclischer Alkylrest oder ein Phenylrest und n = 1 bis 5 ist, oder $(CH_2)_m-Cyc$, wobei Cyc ein isocyclischer oder heterocyclischer Ring mit ein- oder mehrcyclischer Struktur oder ein aromatischer oder heteroaromatischer Ring mit ein- oder mehrcyclischer Struktur und m = 0 bis 5 ist, bedeutet, dadurch gekennzeichnet, dass eine Verbindung der Formel

$$(\frac{1}{\alpha}Me)\,O - CH = C(R) - CN \qquad (2)$$

wobei R dieselbe Bedeutung wie in der Formel (1) hat und Me ein Alkalimetall mit α = 1 oder ein Erdalkalimetall mit α = 2 ist, mit einem Säureadditionssalz des Hydroxylamins der Formel

$$H_2N\,OH \cdot HX \qquad (3)$$

wobei HX eine ein- oder mehrbasische anorganische oder organische Säure bedeutet, bei Temperaturen von −10°C bis 80°C und Drücken zwischen 1 und 5 bar umgesetzt wird.

2. Verfahren nach Anspruch 1, dadurch gekennzeichnet, dass die Säure HX eine Halogenwasserstoffsäure oder Schwefelsäure ist.

3. Verfahren nach Anspruch 2, dadurch gekennzeichnet, dass die Halogenwasserstoffsäure Chlorwasserstoff ist.

4. Verfahren nach einem der beiden Ansprüche 1 oder 2, dadurch gekennzeichnet, dass die Umsetzung in einem polaren Lösungsmittel oder in Gegenwart eines polaren Lösungsmittels erfolgt.

5. Verfahren nach Anspruch 4, dadurch gekennzeichnet, dass die Umsetzung in einem polaren Lösungsmittel erfolgt.

**Claims**

1. Process for the production of 5-aminoisoxazolenes of the formula

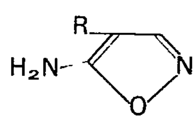

(1)

wherein R denotes a hydrogen atom, straight-chained, branched or cyclic alkyl residues with 1 to 20 carbon atoms, straight-chained or branched residues $-(CH_2)_n-CN$, $-(CH_2)_n-COOR'-$, $-(CH_2)_n-NH_2$, $-(CH_2)_n-OR'$, wherein R' is a straight-chained, branched or cyclic alkyl residue or a phenyl residue and n = 1 to 5, or $(CH_2)_m-Cyc$, wherein Cyc is an isocyclic or heterocyclic ring with mono or polycyclic structure or an aromatic or heteroaromatic ring with mono or polycyclic structure and m = 0 to 5, characterised in that a compound of the formula

$$(\frac{1}{\alpha}Me)\,O - CH = C(R) - CN \qquad (2)$$

wherein R has the same meaning as in formula (1) and Me is an alkali metal, when $\alpha = 1$, or an alkali earth metal, when $\alpha = 2$, is reacted at temperatures of $-10°C$ to $80°C$ and pressures between 1 and 5 bar with an acid addition salt of hydroxylamine of the formula

$$H_2N\,OH \cdot HX \qquad (3)$$

wherein HX denotes a mono or poly basic inorganic or organic acid.

2. Process according to claim 1, characterised in that the acid HX is a hydrogen halide acid or sulphuric acid.

3. Process according to claim 2, characterised in that the hydrogen halide acid is hydrochloric acid.

4. Process according to claim 1 or 2, characterised in that the reaction takes place in a polar solvent or in the presence of a polar solvent.

5. Process according to claim 4, characterised in that the reaction takes place in a polar solvent.

## Revendications

1. Procédé de préparation d'amino-5-isoxazoles de formule

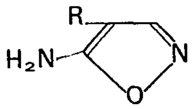

(1)

dans laquelle R représente un atome d'hydrogène, des radicaux alkyles linéaires, ramifiés ou cycliques comportant 1 à 20 atomes de carbone, des radicaux linéaires ou ramifiés $-(CH_2)_n-CN$, $-(CH_2)_n-COOR'$, $-(CH_2)_n-NH_2$, $-(CH_2)_n-OR'$, où R' est un radical alkyle linéaire, ramifié ou cyclique ou bien un radical phényle et n vaut 1 à 5, ou $(CH_2)_m-Cyc$, où Cyc est un noyau isocyclique ou hétérocyclique à structure mono- ou polycyclique ou un noyau aromatique ou hétéroaromatique à structure mono- ou polycyclique et m vaut 0 à 5, caractérisé en ce qu'on fait réagir un composé de formule

$$(\tfrac{1}{\alpha}Me)\,O-CH=C-\underset{\displaystyle R}{\overset{}{C}}N \qquad (2)$$

(dans laquelle R a le même sens que dans la formule (1) et Me est un métal alcalin avec $\alpha = 1$ ou un métal alcalino-terreux avec $\alpha = 2$) avec un sel d'addition d'acide de l'hydroxylamine de formule

$$H_2N\,OH \cdot HX \qquad (3)$$

(dans laquelle HX représente un acide minéral ou organique mono- ou polybasique) à des températures de $-10°C$ à $80°C$ et des pressions comprises entre 1 et 5 bar.

2. Procédé selon la revendication 1, caractérisé en ce que l'acide HX est un hydracide halogéné ou l'acide sulfurique.

3. Procédé selon la revendication 2, caractérisé en ce que l'hydracide halogéné est l'acide chlorhydrique.

4. Procédé selon l'une des revendications 1 ou 2, caractérisé en ce que la réaction s'effectue dans un solvant polaire ou en présence d'un solvant polaire.

5. Procédé selon la revendication 4, caractérisé en ce que la réaction s'effectue dans un solvant polaire.